# FASCICULE DE BREVET EUROPEEN

(11) **EP 4 153 262 B1**
(45) Date de publication et mention de la délivrance du brevet: **03.12.2025**
(21) Numéro de dépôt: 21734397.9
(22) Date de dépôt: 20.05.2021
(51) Int. Cl.: A61M 1/02, A61M 1/36, B01L 3/00

(54) **DISPOSITIF DE SÉPARATION ET DE RÉCUPÉRATION DE FRACTIONS SANGUINES**
VORRICHTUNG ZUR TRENNUNG UND GEWINNUNG VON BLUTFRAKTIONEN
DEVICE FOR SEPARATING AND RECOVERING BLOOD FRACTIONS

(30) Priorité: 20.05.2020 FR 2005199
(43) Date de publication de la demande: 29.03.2023
(73) Titulaire: Cellquest, 25000 Besançon (FR)
(72) Inventeur: WALLART, Guillaume, 25680 MESANDANS (FR)
(74) Mandataire: IP Trust
(86) Numéro de dépôt international: PCT/FR2021/050896
(87) Numéro de publication internationale: WO 2021/234298

(56) Documents cités:
- TW-A- 201 405 127
- US-A- 3 911 918
- US-A1- 2016 129 438

## Description

### Domaine de l'invention

La présente invention concerne le traitement et la séparation automatisés de cellules biologiques telles que celles trouvées dans le sang total, et concerne plus spécifiquement un système fonctionnellement fermé permettant d'extraire certaines populations de cellules telles que les cellules souches hématopoïétiques, pour une utilisation immédiate ou leur mélange avec une solution additive ou une solution de stockage pour des opérations de stockage séparées ultérieures et pour les procédés permettant de réaliser une telle extraction. Le but est notamment d'automatiser l'isolement de leuco-plaquettaire ayant une grande quantité de cellules souches hématopoïétiques et de cellules souches mésenchymateuses.

Il s'agit plus particulièrement du domaine de la séparation et récupération de fractions sanguines par centrifugation, notamment les lymphocytes et d'autres cellules blanches. Notamment, cette séparation à partir d'un prélèvement de sang peut être réalisée en formant en gradient de densité dans un tube à centrifuger, au-dessus d'une couche d'un fluide de densité référencé, par exemple le polyfluorocarbone liquide (Ficoll ^{®}) de densité 1,077.

Une des applications très répandue notamment en Asie concerne la chirurgie esthétique, pour la préparation de plasma riche en plaquettes (PRP), à l'aide d'un tube en forme de sablier et deux ouvertures, soumis à une centrifugation. Le concentré leuco-plaquettaire (buffy coat) est ensuite recueilli au niveau du col du tube.

Le PRP est centrifugé pour éliminer les globules rouges et pour séparer les plaquettes sanguines et le facteur de croissance riche en plaquettes (Buffy coat) riche en plaquettes et en facteurs de croissance. Dans le domaine de la chirurgie, le PRP a pour fonction de reconstruire et de réparer les parties endommagées des vaisseaux sanguins. Il est possible de concentrer les facteurs de croissance favorisant la régénération cutanée ou tissulaire, ou reconstructive (ligaments vivants, tendons) pour le traitement de l'arthrite, des douleurs chroniques au dos, des douleurs pelviennes et des lésions des ligaments de l'épaule et du genou.

Les consommables se présente sous la forme d'un double cône reliées par leurs bases et dont l'un présente sur sa face extérieure un puit prévu destiné à l'alimentation avec le sang prélevé. Par cette même ouverture on prélève après centrifugation la quantité de plasma désirée pour recueillir ensuite les facteurs de croissance présents dans les granules alpha des plaquettes tels que le TGF-β (« transforming growth factor beta »), le VEGF (« vascular endothelial growth factor »), et le PDGF (« platelet-derived growth factor »).

### Etat de la technique

On connait dans l'état de la technique la demande de brevet américain US20160129438 décrivant un godet de collecte de liquide biologique utilisable avec une centrifugeuse pour séparer un liquide biologique en ses éléments constitutifs. Le godet présente la forme d'un sablier à parties supérieure et inférieure évasées et partie centrale rétrécie. Un piston s'insère par coulissement dans la partie inférieure et un orifice latéral est ménagé pour extraction d'un liquide de la partie rétrécie.

On connaît aussi la demande TW201405127A décrivant une structure comprend plusieurs corps. L'un des corps présente un premier compartiment de réception à volume fixe et un second compartiment à volume variable reliés par un rétrécissement définissant un chemin de communication pour faire communiquer le compartiment à volume fixe et le compartiment à volume variable.

On connaît encore la demande de brevet US3911918 décrivant un récipient pour stocker un fluide viable, tel que du sang, construit en une matière plastique flexible formée d'un seul tenant ayant deux compartiments de stockage connectés en communication fluidique par une partie de col de conduit de transfert. L'un des compartiments de stockage a une taille détaillée pour contenir environ 60% d'une quantité prédéterminée de sang avec l'autre compartiment de stockage et la partie du col du conduit de transfert dont la taille est détaillée pour contenir le reste de la quantité prédéterminée de sang. La partie de col du conduit de transfert est constituée d'un matériau qui peut être scellé de sorte que le conteneur peut être divisé en un certain nombre de compartiments autonomes séparés et dans une autre utilisation du conteneur.

### Solution apportée par l'invention

L'invention concerne selon son acception la plus générale un dispositif de séparation et de récupération de fractions sanguines présentant les caractéristiques techniques énoncées par la revendication 1.

Avantageusement, ledit moyen de récupération comprend au moins un flotteur de densité déterminée, positionné dans un canal formé dans l'interface entre lesdites chambres coniques.

Selon une variante, ledit moyen de récupération comprend une vanne à trois voies positionnée dans un canal formé dans l'interface entre lesdites chambres coniques, comportant un embout de sortie.

L'invention concerne aussi un équipement pour la séparation et de récupération de fractions sanguines constitué par une centrifugeuse présentant un plateau caractérisé en ce que ledit plateau présentant au moins un logement pour un dispositif susvisé.

De préférence, ledit plateau présentant au moins un logement pour un dispositif susvisé et comporte un capteur d'images dont le champ de vision correspond à une zone de passage lors de la rotation du segment de communication entre lesdites chambres et une partie adjacente au moins de chacune desdites deux chambres, ledit plateau comportant un moyen de commande de la position de la vanne du dispositif déposé sur ledit plateau.

Avantageusement, il comporte en outre un calculateur commandant ledit moyen de commande en fonction de l'évolution des images acquises par ledit capteur d'images.

### Description détaillée de l'invention

La présente invention sera mieux comprise à la lecture de la description qui suit, se référant aux dessins annexés relative à un exemple non limitatif de réalisation, où :

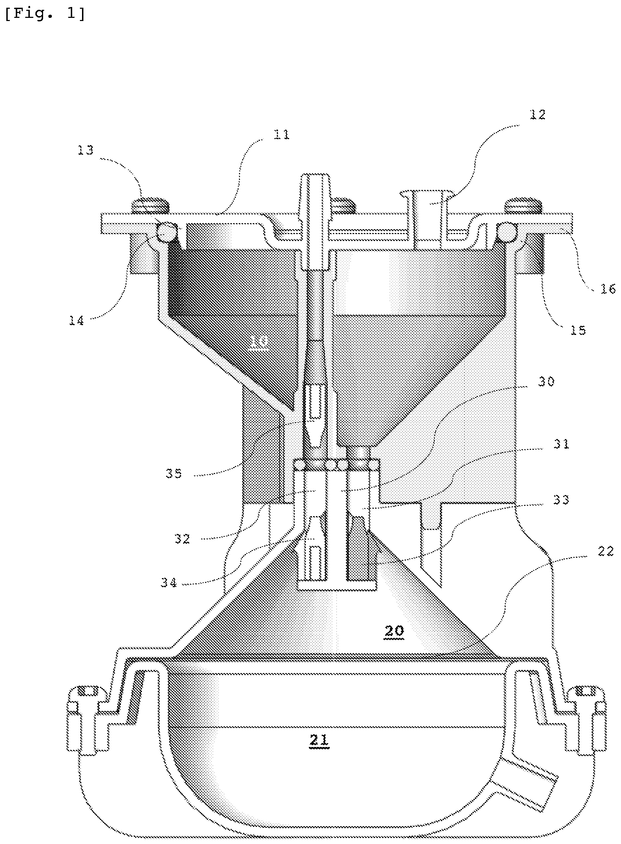

[Fig. 1] la figure 1 représente une vue en coupe longitudinal d'un dispositif selon l'invention
[Fig. 2] la figure 2 représente une vue en coupe longitudinal d'une variante du dispositif selon l'invention avec une vanne trois voies
[Fig. 3] la figure 3 représente une vue en coupe longitudinal de ladite variante avec une vanne trois voies à l'étape de chargement du sang
[Fig. 4] la figure 4 représente une vue en coupe longitudinal de ladite variante avec une vanne trois voies à l'étape de centrifugation
[Fig. 5] la figure 5 représente une vue en coupe longitudinal de ladite variante avec une vanne trois voies à l'étape de séparation
[Fig. 6] la figure 6 représente une vue en coupe longitudinal de ladite variante avec une vanne trois voies à l'étape de décélération
[Fig. 7] la figure 7 représente une vue en coupe longitudinal de ladite variante avec une vanne trois voies à l'étape de rotation du maneton
[Fig. 8] la figure 8 représente une vue en coupe longitudinal de ladite variante avec une vanne trois voies à l'étape de reprise de la décélération
[Fig. 9] la figure 9 représente une vue en coupe longitudinal de ladite variante avec une vanne trois voies à l'étape de remise en communication des deux cônes du dispositif.

### Premier contexte applicatif de l'invention

L'invention sera décrite en référence des exemples de réalisation qui suivent dans le contexte plus spécifique des thérapies cellulaires par des composants sanguins.

L'invention décrite ci-après concerne un dispositif, de préférence à usage unique, destiné à un système automatisé permettant de traiter et de se concentrer sur les cellules souches sans perte ni altération de leur fonctionnalité, notamment pour la greffe de cellules souches progénitrices hématopoïétiques.

La fraction riche en cellules souches est isolée à partir du sang périphérique prélevé dans le système circulatoire.

Pour isoler les cellules souches de la couche leuco-plaquettaire on utilise des produits à gradient de densité tels que ceux disponibles sous les noms Ficoll et Percoll (marques commerciales). Le produit à gradient de densité est d'abord introduit dans le dispositif de traitement, suivi par l'introduction de sang total, puis un composant du fluide biologique est séparé par centrifugation et collecté par pipetage par exemple.

### Description physique du dispositif

Le dispositif de centrifugation selon l'invention est formé par une pièce moulée en matière plastique présentant une forme générale de sablier ou de bicone, avec :
- une partie conique périphérique (10) présentant à son extrémité périphérique une grande base fermée par un couvercle (11) présentant un orifice d'alimentation (12)
- une partie conique centrale (20) dont l'extrémité centrale est séparée d'un fond concave (21) par une membrane (22) élastique
- un manchon de liaison (30) entre la base de la partie conique périphérique (10) et la base de la partie conique centrale (20).

Le couvercle (11) de la partie conique périphérique (10) présente à sa partie centrale une nervure périphériques (13) venant s'appuyer sur un joint annulaire (14) logé dans une gouttière périphérique (15) prévue à la bordure périphérique de la partie conique (10). Le rebord périphérique (16) forme un élément d'accrochage dans une chambre d'une centrifugeuse non représentée.

Le manchon de liaison (30) présente deux canaux (31, 32) débouchant à l'intérieur de la partie conique inférieur (20). Le premier canal (31) met en communication le fond de la partie conique périphérique (10) avec la partie conique centrale (20). Il convient un premier flotteur (33) de densité D1.

Le deuxième canal (32) traverse la partie conique périphérique (10) est débouche à l'extérieur du couvercle (11). Il enferme un premier flotteur (34) de densité D2 et un deuxième flotteur (35) de densité D3.

La membrane (22) est une membrane déformable élastiquement silicone biocompatible d'une élasticité d'environ 450% et d'une épaisseur comprise entre 1 et 2 millimètres, et un diamètre de 50 millimètres. Il assure un bord périphérique torique enserrés dans des gorges annulaires prévus sur la partie frontale de la partie centrale (20) et un couvercle de fermeture.

Le diamètre supérieur des parties coniques périphérique (10) et centrale (20) est de 50 millimètres, et le diamètre intérieur du canal de communication est compris entre 1 et 1,5 millimètres.

### Description d'une variante de réalisation vanne à trois voies

### Description de la centrifugeuse selon la deuxième variante

Le dispositif de centrifugation selon cette variante de réalisation est également formé par une pièce moulée en matière plastique présentant une forme générale de sablier ou de bicone, avec :
- une partie conique périphérique (10) fermée par un couvercle (11) présentant un orifice d'alimentation (12)
- une partie conique centrale (20) dont l'extrémité centrale est séparée d'un fond concave (21) par une membrane (22) élastique
- un manchon de liaison (30) entre la base de la partie conique périphérique (10) et la base de la partie conique centrale (20).

Le manchon de liaison (30) présente un canal (36). Ce canal (36) met en communication le fond de la partie conique périphérique (10) avec la partie conique centrale (20). Il est muni d'un maneton commandant une vanne trois voies (37) qui, selon sa position commande :
- l'écoulement de fluide entre les deux parties coniques (10, 20)
- l'écoulement de fluide entre la partie conique périphérique (10) et un tube de prélèvement (38)
- la fermeture du canal (37).

La membrane (22) est une membrane déformable élastiquement silicone biocompatible d'une élasticité d'environ 450% et d'une épaisseur comprise entre 1 et 2 millimètres, et un diamètre de 50 millimètres. Elle présente une bordure périphérique torique (23) engagée entre une gorge annulaire (24) prévue sur la bordure frontale de la partie conique (20) et une gorge (25) formée dans le fond concave (21). Le bord de cette gorge (25) présente une nervure (26) assurant le maintien par clipsage de la partie conique (20).

### Description fonctionnelle

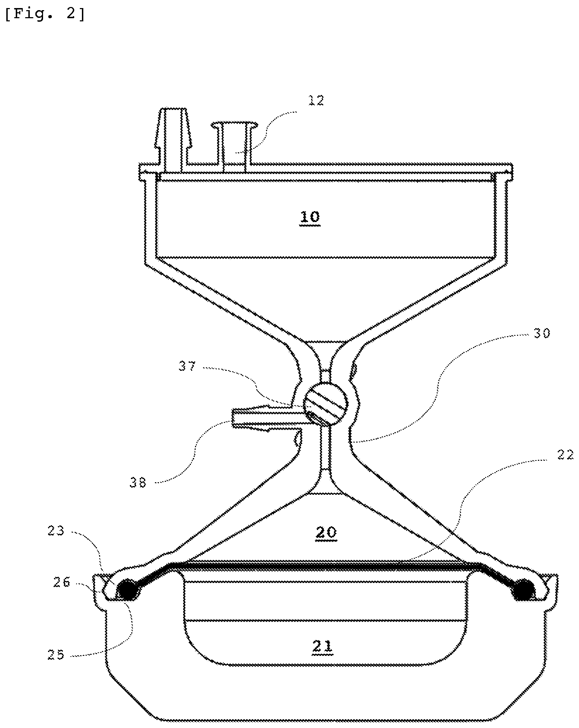

Les figures 2 à 9 illustre une séquence de centrifugation d'un échantillon de sang.

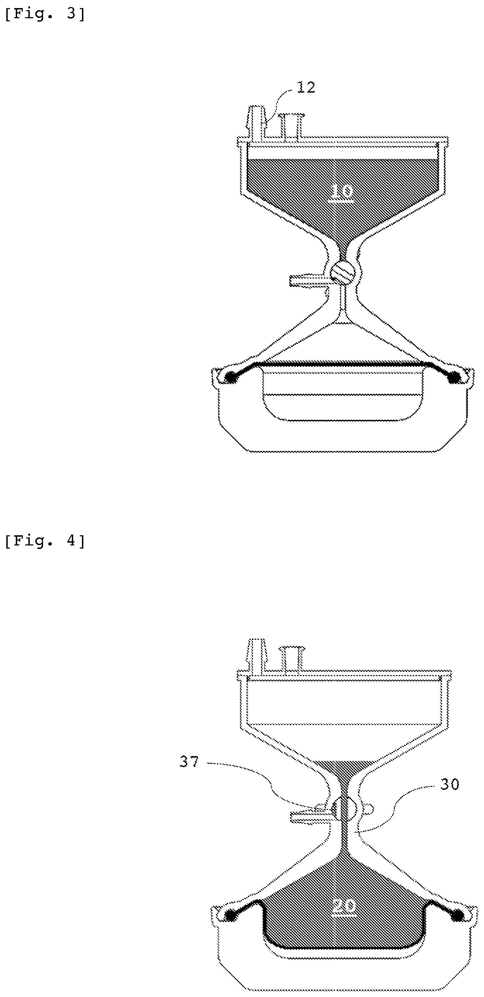

L'échantillon de sang est transféré depuis une poche stérile vers la partie conique périphérique (10) par un tube stérile la reliant à l'embout (12) de remplissage (figure 3).

On place ensuite le dispositif dans une centrifugeuse, la partie conique (10) étant placée du côté du centre de la centrifugeuse et la partie conique (20) du côté périphérique. La vanne trois voies (37) est placée en position passante, pour permettre au sang contenu dans la partie conique (10) de s'écouler vers la partie conique (20) sous l'effet de la force centrifuge (figure 4).

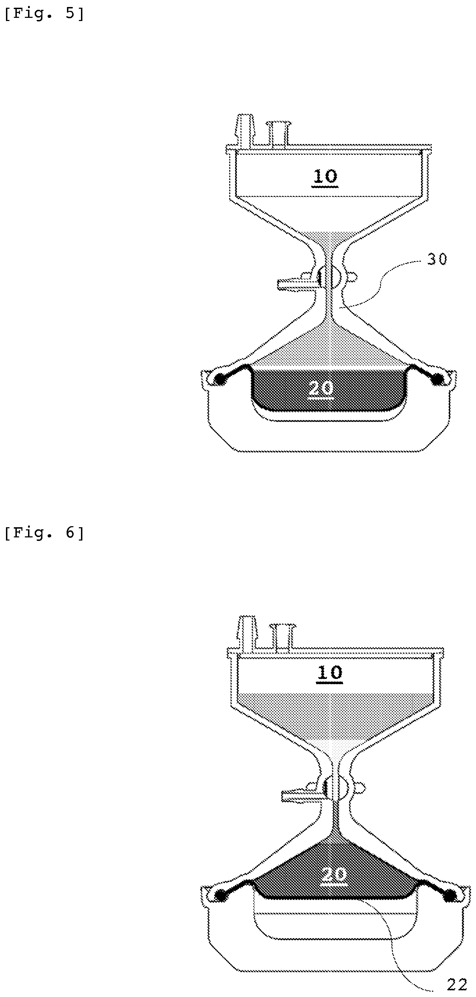

La centrifugeuse est ensuite arrêtée pour permettre aux différents composés du sang de se répartir en fonction de leur densité, de manière croissante les globules rouges, les globules blancs, les plaquettes et le plasma (figure 5).

On procède ensuite à une décélération lente pour remonter la couche d'intérêt juste sous la zone de tri. Une fois la couche d'intérêt arrivée sur zone, passage en vitesse de centrifugation constante : les couches s'immobilisent et la membrane élastique équilibre la pression (figure 6).

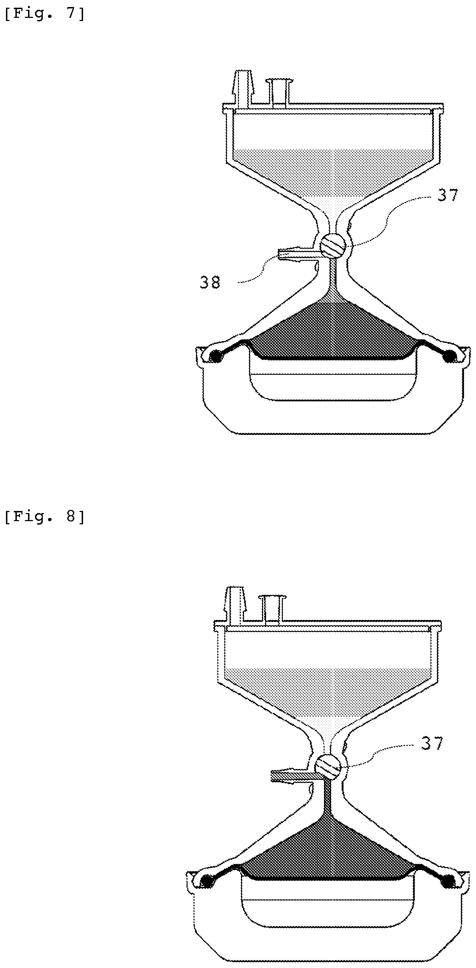

On bascule alors la vanne à trois voies (37) en position de mise en communication de l'embout de prélèvement (38) pour permettre le prélèvement des plaquettes (figure 7).

On bascule alors la vanne à trois voies (37) en position de mise en communication de l'embout de prélèvement (38) pour permettre le prélèvement des plaquettes (figure 7).

On remet la centrifugeuse en décélération lente : la couche d'intérêt est évacuée par l'embout de sortie latérale (38). Dès que la couche de plaquette est récupérée, on commande le retour en vitesse constante (figure 8).

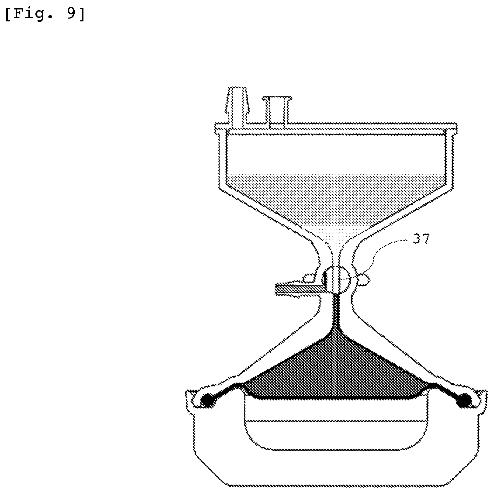

Le maneton commandant la vanne trois voies est ensuite replacé en position passante. La couche d'intérêt est isolée (figure 9).

## Revendications

1. - Dispositif de séparation et de récupération de fractions sanguines par deux chambres coniques (10, 20) communiquant par leurs bases, la première chambre (10) présentant un conduit d'alimentation du fluide à traiter et des moyens de récupération d'au moins un composant
**Caractérisé en ce que**
La seconde chambre (20) comprenant une membrane souple (22) déformable élastiquement séparant transversalement un espace débouchant dans le conduit de communication avec ladite seconde chambre (20), et un fond concave (21), de volume variable en fonction de la déformation de ladite membrane.

2. - Dispositif selon la revendication 1 **caractérisé en ce que** ledit moyen de récupération comprend au moins un flotteur de densité déterminée, positionné dans un canal formé dans l'interface entre lesdites chambres coniques (10, 20).

3. - Dispositif selon la revendication 1 **caractérisé en ce que** ledit moyen de récupération comprend une vanne à trois voies positionnée (37) dans un canal (36) formé dans l'interface entre lesdites chambres coniques, comportant un embout de sortie.

4. - Équipement pour la séparation et de récupération de fractions sanguines constitué par une centrifugeuse présentant un plateau **caractérisé en ce que** ledit plateau présentant au moins un logement pour un dispositif conforme à l'une au moins des revendications 1 à 3.

5. - Équipement pour la séparation et de récupération de fractions sanguines selon la revendication précédente, **caractérisé en ce que** ledit plateau présentant au moins un logement pour un dispositif conforme à la revendication 3 et ce qu'il comporte un capteur d'images dont le champ de vision correspond à une zone de passage lors de la rotation du segment de communication entre lesdites chambres et une partie adjacente au moins de chacune desdites deux chambres, ledit plateau comportant un moyen de commande de la position de la vanne du dispositif déposé sur ledit plateau.

6. - Équipement pour la séparation et de récupération de fractions sanguines selon la revendication précédente, **caractérisé en ce qu'**il comporte en outre un calculateur commandant ledit moyen de commande en fonction de l'évolution des images acquises par ledit capteur d'images.

## Patentansprüche

1. Vorrichtung zur Trennung und Gewinnung von Blutfraktionen mittels zweier konischer Kammern (10, 20), die über ihre Böden miteinander verbunden sind, wobei die erste Kammer (10) einen Zufuhrkanal für die zu behandelnde Flüssigkeit und Mittel zur Gewinnung von mindestens einer Komponente aufweist,
**dadurch gekennzeichnet, dass**
die zweite Kammer (20) eine elastisch verformbare flexible Membran (22), die einen in die Leitung zur Verbindung mit der zweiten Kammer (20) mündenden Raum quer trennt, und einen konkaven Boden (21) mit einem Volumen umfasst, das sich in Abhängigkeit von der Verformung der Membran verändert.

2. Vorrichtung nach Anspruch 1, **dadurch gekennzeichnet, dass** das Mittel zur Gewinnung mindestens einen Schwimmer mit bestimmter Dichte umfasst, der in einem Kanal positioniert ist, der in der Schnittstelle zwischen den konischen Kammern (10, 20) ausgebildet ist.

3. Vorrichtung nach Anspruch 1, **dadurch gekennzeichnet, dass** das Mittel zur Gewinnung ein Dreiwegeventil (37) umfasst, das in einem Kanal (36) positioniert ist, der in der Schnittstelle zwischen den konischen Kammern ausgebildet ist und einen Auslassstutzen beinhaltet.

4. Vorrichtung zur Trennung und Gewinnung von Blutfraktionen, bestehend aus einer Zentrifuge, die eine Platte aufweist, **dadurch gekennzeichnet, dass** die Platte mindestens eine Aufnahme für eine Vorrichtung nach mindestens einem der Ansprüche 1 bis 3 aufweist.

5. Vorrichtung zur Trennung und Gewinnung von Blutfraktionen nach dem vorstehenden Anspruch, **dadurch gekennzeichnet, dass** die Platte mindestens eine Aufnahme für eine Vorrichtung nach Anspruch 3 aufweist und dass sie einen Bildsensor umfasst, dessen Sichtfeld einem Durchgangsbereich beim Drehen des Verbindungssegments zwischen den Kammern und einem Abschnitt entspricht, der zumindest an jede der beiden Kammern angrenzt, wobei die Platte ein Mittel zur Steuerung der Position des Ventils der auf der Platte abgelegten Vorrichtung umfasst.

6. Vorrichtung zur Trennung und Gewinnung von Blutfraktionen nach dem vorstehenden Anspruch, **dadurch gekennzeichnet, dass** sie weiter einen Rechner umfasst, der das Mittel zur Steuerung in Abhängigkeit von der Entwicklung der vom Bildsensor erfassten Bilder steuert.

## Claims

1. Device for separating and recovering blood fractions by means of two conical chambers (10, 20) communicating via their bases, the first chamber (10) having a duct for supplying the fluid to be treated and means for recovering at least one component,
**characterised in that**
the second chamber (20) comprising an elastically deformable flexible membrane (22) which transversely separates a space opening into the duct for communicating with the second chamber (20), and a concave bottom (21), having a volume which varies according to the deformation of said membrane.

2. Device according to claim 1, **characterised in that** said recovery means comprises at least one float of determined density, positioned in a channel formed in the interface between said conical chambers (10, 20).

3. Device according to claim 1, **characterised in that** said recovery means comprises a three-way valve (37) positioned in a channel (36) formed in the interface between said conical chambers, comprising an outlet tip.

4. Equipment for separating and recovering blood fractions constituted by a centrifuge having a plate **characterised in that** said plate having at least one housing for a device according to at least one of claims 1 to 3.

5. Equipment for separating and recovering blood fractions according to the preceding claim, **characterised in that** said plate having at least one housing for a device according to claim 3, and **in that** it comprises an image sensor, the viewing field of which corresponds to a passage zone during the rotation of the communication segment between said chambers and at least one adjacent part of each of said two chambers, said plate comprising a means for controlling the position of the valve of the device placed on said plate.

6. Equipment for separating and recovering blood fractions according to the preceding claim, **characterised in that** it further comprises a computer, controlling said control means according to the development of the images acquired by said image sensor.
